# EUROPEAN PATENT SPECIFICATION

(11) **EP 2 758 129 B1**
(45) Date of publication and mention of the grant of the patent: **14.12.2016**
(21) Application number: 12829018.6
(22) Date of filing: 31.08.2012
(51) Int. Cl.: A61N 5/06, A61N 5/067, A61K 39/39, A61K 39/12, A61K 39/00

(54) **LASER ADJUVANTS FOR ENHANCING IMMUNE RESPONSE**
LASERADJUVANTIEN ZUR STEIGERUNG DER IMMUNANTWORT
ADJUVANTS LASER POUR AMÉLIORATION DE RÉPONSE IMMUNITAIRE

(30) Priority: 01.09.2011 US 201161530296 P
(43) Date of publication of application: 30.07.2014
(73) Proprietor: The General Hospital Corporation, Boston, MA 02114 (US)
(72) Inventor: KASHIWAGI, Satoshi, Boston, Massachusetts 02114 (US); POZNANSKY, Mark, Charlestown, Massachusetts 02129 (US)
(74) Representative: Donald, Jenny Susan
(86) International application number: PCT/US2012/053282
(87) International publication number: WO 2013/033496

(56) References cited:
- WO-A2-2009/044272
- RU-A- 2005 127 615
- RU-C2- 2 410 735
- US-A1- 2003 078 499
- MOSKVINA S.V. ET AL.: '''Firma ''Tekhnika''' NIZKOINTENSIVNAYA LAZERNAYA TERAPIYA. 2000, MOSKVA, pages 35 - 36, 102-103, 152, 154-157, XP008173678

### SEQUENCE LISTING

<110> The General Hospital Corporation
<120> Laser Adjuvants for Enhancing Immune
   Response
<130> 29539-0044WO1
<150> US 61/530,296
   <151> 2011-09-01
<160> 4
<170> FastSEQ for Windows Version 4.0
<210> 1
   <211> 8
   <212> PRT
   <213> Artificial Sequence
<220>
   <223> nucleoprotein MHC class I OVA-specific peptide
<400> 1
   Ser Ile Ile Asn Phe Glu Lys Leu
   1 5
<210> 2
   <211> 9
   <212> PRT
   <213> Artificial Sequence
<220>
   <223> nucleoprotein MHC class I influenza-specific peptide
<400> 2
   Ala Ser Asn Glu Asn Met Glu Thr Met
   1 5
<210> 3
   <211> 17
   <212> PRT
   <213> Artificial Sequence
<220>
   <223> nucleoprotein MHC class II OVA-specific peptide
<400> 3
   Ile Ser Gln Ala Val His Ala Ala His Ala Glu Ile Asn Glu Ala Gly
   1 5 10 15
   Arg
<210> 4
   <211> 15
   <212> PRT
   <213> Artificial Sequence
<220>
   <223> nucleoprotein MHC class II influenza-specific peptide
<400> 4
   Gln Val Tyr Ser Leu Ile Arg Pro Asn Glu Asn Pro Ala His Lys
   1 5 10 15

## Description

### FEDERALLY SPONSORED RESEARCH OR DEVELOPMENT

This invention was made with Government support under Grant No. N66001-10-1-2132 awarded by the Department of Defense. The Government has certain rights in the invention.

### TECHNICAL FIELD

This disclosure relates to methods for enhancing an immune response to a vaccine using co-administration of a laser.

### BACKGROUND

Current vaccines are generally highly-targeted recombinant molecules, poorly immunogenic, and require enhancement with immunologic adjuvants (Harandi AM, Davies G, Olesen OF. (2009) Expert Rev Vaccines 8(3): 293-8; Leroux-Roels G. (2010)Vaccine 28 Suppl 3: C25-36; Perrie Y, Mohammed AR, Kirby DJ, McNeil SE, Bramwell VW. (2008) Int J Pharm 364(2): 272-80; Nichol KL. (2008) Vaccine 26 Suppl 4: D17-22; Reed SG, Bertholet S, Coler RN, Friede M. (2009) Trends Immunol 30(1): 23-32). The development of safe and potent immunologic adjuvants is essential for current vaccine practice, but has been frustrating for numerous reasons. Despite a proliferation of possible adjuvant candidates, few have demonstrated sufficient effectiveness and safety in their development. The FDA has approved only two immunologic adjuvants over the past 80 years, mainly due to a concern for side effects (Leroux-Roels G. (2010); Reed et al. (2009); Aguilar JC, Rodriguez EG. (2007) Vaccine 25(19): 3752-62; Coffman RL, Sher A, Seder RA. (2010) Immunity 33(4): 492-503). The stimulatory nature of chemicals of conventional adjuvant candidates contributes to their ability to over-activate the immune response, resulting in undesirable side effects and toxicity to the biological system (Leroux-Roels G. (2010); Perrie et al. (2008); Reed et al. (2009); Israeli E, Agmon-Levin N, Blank M, Shoenfeld Y. (2009) Lupus 18(13): 1217-25). Furthermore, this uncontrollable stimulation to the immune system generally leads to limited efficacy. The most prevalent immunologic adjuvant, aluminium hydroxide salt, elicits little cell-mediated immune response and is poorly effective to certain vaccines (Leroux-Roels G. (2010); Reed et al. (2009). This is a common issue among potential, but not yet clinically acceptable, adjuvant candidates.

The skin is the first-line of defense against pathogens, and the immune system's surveillance of the integrity of this structure is essential for the maintenance of life (Nestle FO, Di Meglio P, Qin JZ, Nickoloff BJ. (2009) Nat Rev Immunol 9(10): 679-91; Kaplan DH. (2010) Trends Immunol 31(12): 446-51; Abraham SN, St John AL. (2010) Nat Rev Immunol 10(6): 440-52). Therefore, the skin-based immune system can be effectively primed and ready to respond to pathogens through vaccination at this surface (Wahl M, Hermodsson S. (1987) Scand J Infect Dis 19(6): 617-21; Vankerckhoven V, Van Damme P. (2010) Expert Opin Drug Deliv 7(9): 1109-25; Sticchi L, Alberti M, Alicino C, Crovari P. (2010) J Prev Med Hyg 51(1): 7-14; Nicolas JF, Guy B. (2008) Expert Rev Vaccines 7(8): 1201-14; Prausnitz MR, Mikszta JA, Cormier M, Andrianov AK. (2009) Curr Top Microbiol Immunol 333: 369-93; Lambert PH, Laurent PE. (2008) Vaccine 26(26): 3197-208). An intradermal route of vaccination has recently been evaluated, and it appears to be more effective than the conventional intramuscular route common to many vaccinations, including influenza and hepatitis B (Vankerckhoven et al. (2010); Nicolas JF, Guy B. (2008); Prausnitz et al. (2009); Lambert et al. (2008) ; Sangare L, Manhart L, Zehrung D, Wang CC. (2009) Vaccine 27(12): 1777-86). The combination of a skin-based vaccination and adjuvant has the potential to yield the highest effectiveness.

### SUMMARY

The present disclosure is based, at least in part, on the discovery that specific wavelengths (e.g., near-infrared, e.g., about 1064 nm) of non-tissue damaging laser light (e.g., less than 1 W in power), delivered continuously over short periods of time (e.g., less than about 4 minutes, e.g., 10 seconds to 2 minutes, e.g., 30 to 90 seconds) on a small region on the skin (e.g., less than 0.5 cm²), can stimulate immune responses to vaccines. Thus, the methods described herein include the use of such laser adjuvants in combination with vaccine administration.

In a first aspect, the disclosure provides methods for enhancing a protective immune response to an antigen (e.g., a vaccine) in a subject. The method includes exposing the skin of the subject to continuous-wave radiation at a wavelength of 1064 nm; and administering to the subject a dose of the antigen, wherein the antigen is administered intradermally, into or immediately adjacent to, the skin exposed to the irradiation.

In a further aspect, the disclosure provides methods for enhancing a protective immune response to an antigen in a subject. The methods include exposing the skin of the subject to continuous-wave radiation at a wavelength of between 1050 nm and 1080 nm, in a total dose of near-infrared radiation of between 25 J and 1200 J; and administering to the subject a dose of the antigen, wherein the antigen is administered intradermally, into or immediately adjacent to, the skin exposed to the irradiation. In some embodiments, a central wavelength of the near-infrared radiation is 1064 nm.

In a further aspect, the disclosure provides methods for enhancing a protective immune response to an antigen in a subject. The methods include exposing the skin of the subject to pulsed radiation at a wavelength of between 1050 nm and 1080 nm, in a total dose of near-infrared radiation of between 25 J and 1400 J; and administering to the subject a dose of the antigen, wherein the antigen is administered intradermally, into or immediately adjacent to, the skin exposed to the irradiation. In some embodiments, a central wavelength of the near-infrared radiation is 1064 nm.

In some embodiments of the methods described herein, the antigen comprises nucleic acid or protein. In some embodiments of the methods described herein, the antigen is viral, bacterial, fungal, unicellular or multicellular parasite or a portion thereof. In some embodiments of the methods described herein, the antigen is an influenza virus or a portion thereof. In some embodiments of the methods described herein, the antigen is from polio, hepatitis A, hepatitis B, hepatitis c, influenza, tuberculosis (Bacillus Calmette-Guérin (BCG)), measles, tetanus toxoid, tick-bourne encepalisis, yellow fever, diphtheria-tetenus-pertussis, human papilloma virus, multivalent meningitis conjugate vaccine, pneumococcal conjugate vaccine or rabies pathogen..

In some embodiments of the methods described herein, a total exposure time of the subject is between 10 seconds and 300 seconds, e.g., between 10-90 seconds, e.g., 30-60 seconds.

In some embodiments of the methods described herein, a cross-sectional intensity profile of the radiation is substantially parabolic in shape. In some embodiments of the methods described herein, the average irradiance is between about 1 W/cm² and about 5 W/cm². In some embodiments of the methods described herein, the average irradiance is about 1 W/cm².

In some embodiments of the methods described herein, the radiation comprises laser radiation, e.g., from a neodymium-doped yttrium aluminum garnet (Nd:YAG) laser. In some embodiments of the methods described herein, the radiation comprises radiation produced by a non-laser source.

Unless otherwise defined, all technical and scientific terms used herein have the same meaning as commonly understood by one of ordinary skill in the art to which this invention belongs. Methods and materials are described herein for use in the present disclosure; other, suitable methods and materials known in the art can also be used. The materials, methods, and examples are illustrative only and not intended to be limiting.

The invention is defined in the claims. Other embodiments are merely exemplary.

Other features and advantages of the invention will be apparent from the following detailed description and figures, and from the claims.

### DESCRIPTION OF DRAWINGS

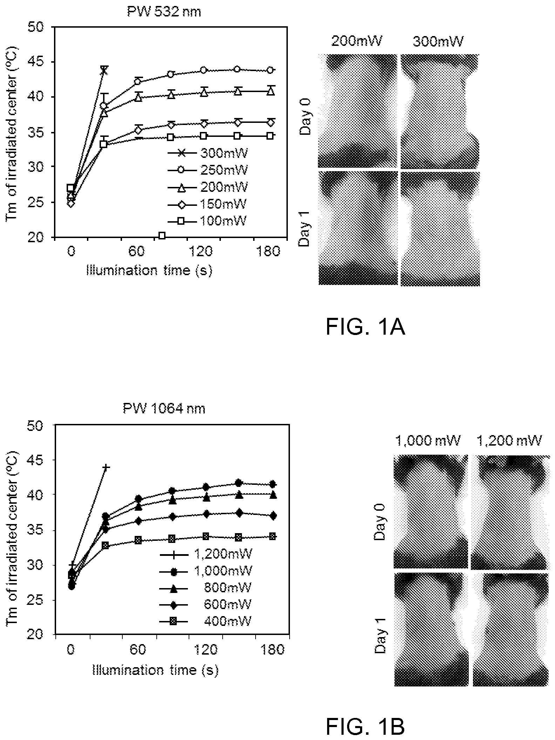

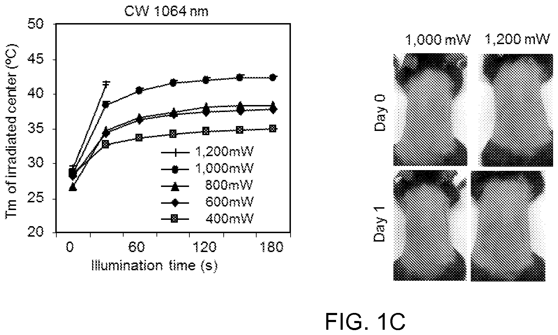

FIG. 1A-C are a set of three graphs and photographic images showing the effect of the laser adjuvant on skin tissue. FIG. 1A-C (left columns) demonstrate surface skin temperatures following continuous wave (CW) or nanosecond-pulsed (PW) 1064 nm or PW 532nm laser illumination on four areas of the shaved and depilated back skin for up to three min. Error bars show means ± s.e.m. FIG. 1A-C (right columns) are photographic images of at 0 and 24 hours after cutaneous laser illumination. Arrow heads, visible skin damage. n =1-4 for each group.

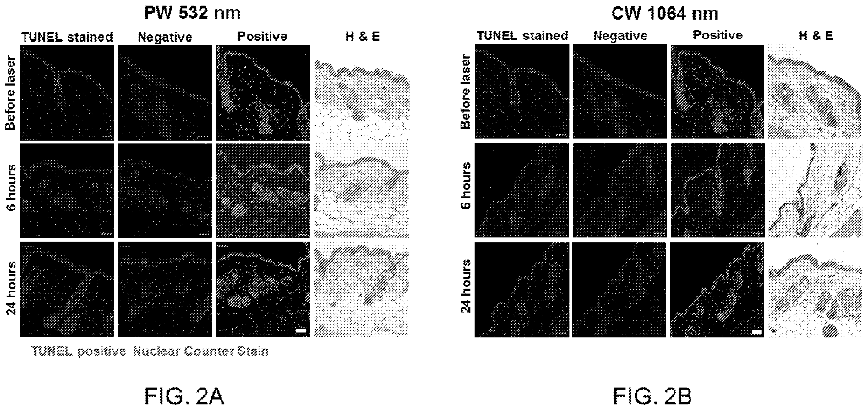

FIG. 2A-B are a set of photomicrographs showing microscopic damage by the non-tissue damaging parameter of the laser adjuvant. Representative time-course images of mouse skin histology with 4-min PW 532 nm (200 mW) (FIG. 2A) and 4-min CW 1064 nm (1000 mW) (FIG. 2B) laser illumination are shown. Skin damage was evaluated at 0, 6, 24 hours after the laser illumination by histology. The left 3 columns show images of TUNEL stained skin tissue. Apoptotic cells were identified in red. The right most columns show images of hematoxylin-Eosin stained skin tissue. The bar indicates 50 µm. (a-c) n = 2-4 for each group.

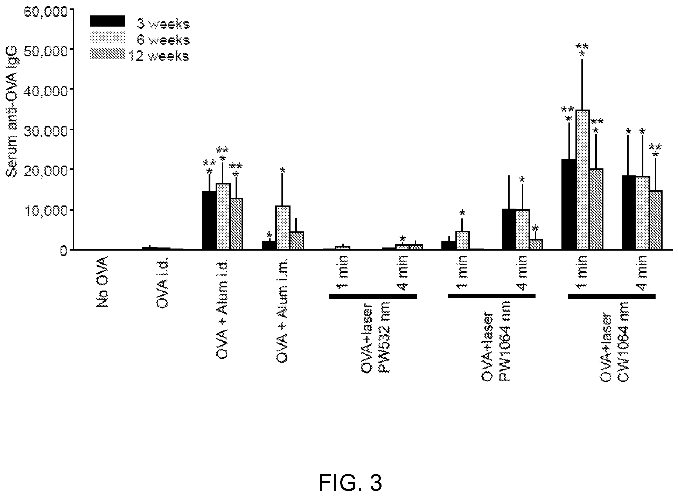

FIG. 3 is a graph showing the serum ovalbumin- (OVA-) specific IgG titers at 3, 6, and 12 weeks after vaccination by intradermal (i.d.) or intramuscular (i.m.) injection of OVA with or without laser illumination or alum. Experimental and control groups: non-vaccination (n = 33), non-adjuvant (i.d. injection of OVA only, n = 19), i.d.-injected alum (i.d. injection of OVA and alum, n = 15), i.m.-injected alum (i.m. injection of OVA and alum, n = 6), 1-min pulsed (PW) 532 nm laser-treated (i.d. injection of OVA and with the laser treatment, n = 12), 4-min PW 532 nm laser-treated (n = 11), 1-min PW 1064 nm laser-treated (i.d. injection of OVA and with the laser treatment, n = 5), 4-min PW 1064 nm laser-treated, n = 6), 1-min continuous wave (CW) 1064 nm laser-treated (i.d. injection of OVA and with the laser treatment, n = 6), 4-min CW 1064 nm laser-treated groups, n = 6). Error bars show means ± s.e.m. *P < 0.05 as compared to the non-adjuvant, **P < 0.05 to PW 532 nm laser-treated (to the equivalent exposure time) groups.

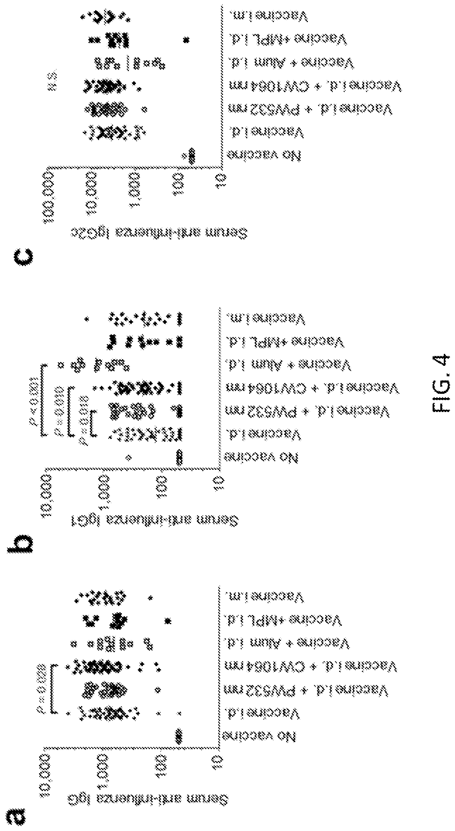

FIG. 4 is a set of graphs showing the effect of laser adjuvant on the humoral immune response to vaccination. FIG. 4a-c demonstrate IgG subclass titers in pre-challenge status (4 weeks after vaccination). Experimental and control groups: non-vaccination in pre-challenge status (a-c: n = 38), non-adjuvant (i.d. injection of influenza vaccine, a-c: n = 49), 4-min PW 532 nm laser-treated (i.d. injection of influenza vaccine with the laser treatment, a-c: n = 26), 1-min CW 1064 nm laser-treated (a-c: n = 52), i.d.-injected alum (a-c: n = 12), i.d.-injected MPL (a-c: n = 12), i.m.-injected groups (i.m. injection of influenza vaccine, a-c: n = 24).

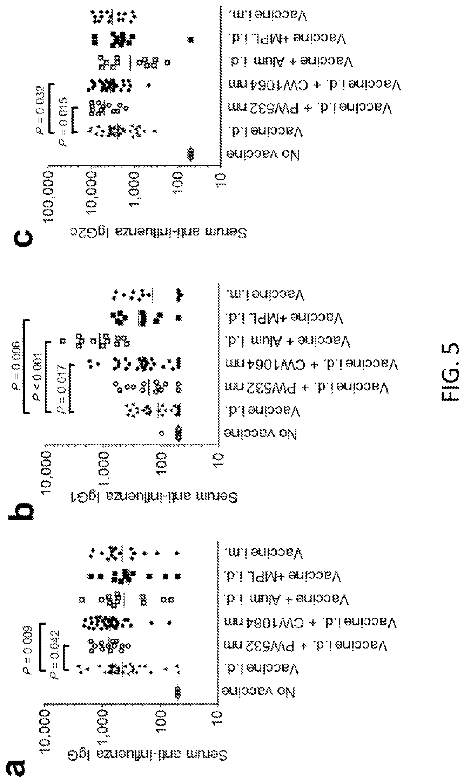

FIG. 5 is a set of graphs showing the effect of laser adjuvant on the humoral immune response to vaccination. FIG. 5a-c demonstrate serum influenza-specific IgG isotype titers in post-challenge status (4 days after challenge). Experimental and control groups: non-vaccination in post-challenge (a-c: n = 25) a, non-adjuvant (i.d. injection of influenza vaccine, a-c: n = 31), 4-min PW 532 nm laser-treated (i.d. injection of influenza vaccine with the laser treatment, a-c: n = 13), 1-min CW 1064 nm laser-treated (a-c: n = 34), i.d.-injected alum (a-c: n = 12), i.d.-injected MPL (a-c: n = 12), i.m.-injected groups (i.m. injection of influenza vaccine, a-c: n = 16), respectively.

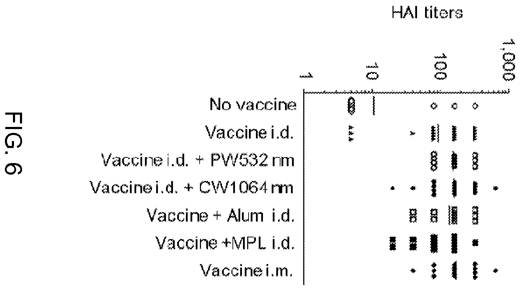

FIG. 6 is a graph demonstrating serum HAI titers after challenge. Experimental and control groups: non-vaccination (n = 14), non-adjuvant (n = 21), PW 532 nm laser-treated (n = 13), CW 1064 nm laser-treated (n = 24), i.d.-injected alum (n = 12), i.d.-injected MPL (n = 12), i.m.-injected groups (n = 16) in post-challenge status.

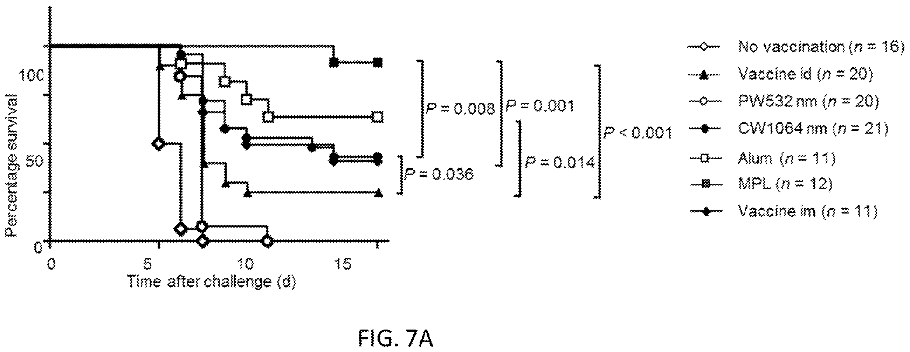

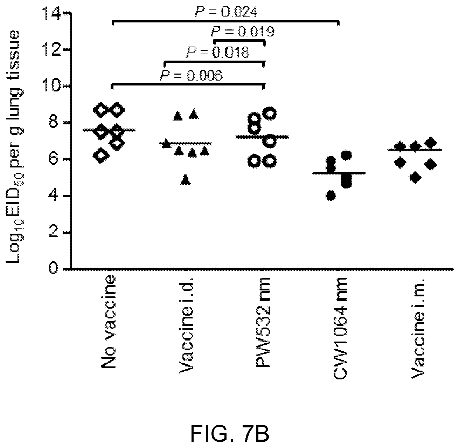

FIG. 7A-B are a set of graphs showing the effect of the laser adjuvant on disease protection. (A) Kaplan-Meier survival plots of vaccinated mice upon the lethal challenge. experimental and control groups: non-vaccination (n = 16), non-adjuvant (n = 20), pulsed 532 nm laser-treated (n = 20), CW 1064 nm laser-treated (n = 21), i.d.-injected alum (n = 11), i.d.-injected MPL (n = 12), i.m.-injected groups (n = 11). (B) The 50% egg infectious dose (EID50) was determined by serial titration of lung homogenate in eggs at 4 days after challenge. Experimental and control groups: non-vaccination (n = 6), non-adjuvant (n = 7), PW 532 nm laser-treated (n = 6), CW 1064 nm laser-treated (n = 6), i.m.-injected groups (n = 6).

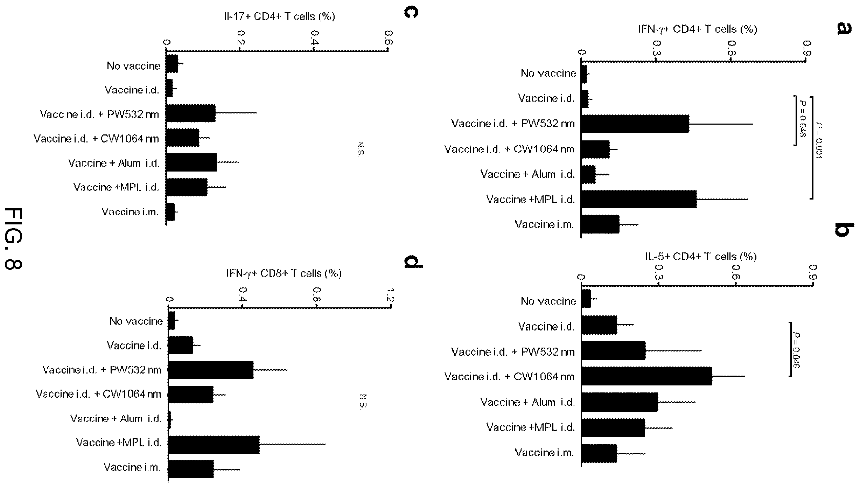

FIG. 8 is a set of graphs showing the effect of the laser adjuvant on a systemic anti-influenza T helper type 1 (Th1)-immune response. (a) Systemic T helper type 1 (Th1)-immune responses after challenge were determined from 5 h-splenocyte culture after isolation from vaccinated and unvaccinated mice at 4 days after challenge and re-stimulation with a nucleoprotein (NP) MHC class II influenza-specific peptide. Percentage of CD4+IFN-γ+ cells is shown. (b) T helper type 2 (Th2)-immune responses. Percentage of CD4+IL-5+ cells is shown. (c) T helper type 17 (Th17)-immune responses. Percentage of CD4+IL-17+ cells is shown. (d) Systemic cytotoxic T cell-immune response were determined from 5 h-splenocyte culture after isolation from vaccinated and unvaccinated mice at 4 days after challenge and re-stimulation with a nucleoprotein (NP) MHC class I influenza-specific peptide. Percentage of CD8+IFN-γ+ cells is shown. Experimental and control groups; non-vaccination (n = 16), non-adjuvant (n = 18), PW 532 nm laser-treated (n = 13), CW 1064 nm laser-treated (n = 22), i.d.-injected alum (n = 10), i.d.-injected MPL (n = 12), i.m.-injected groups (n = 13) in post-challenge status. Error bars show means ± s.e.m.

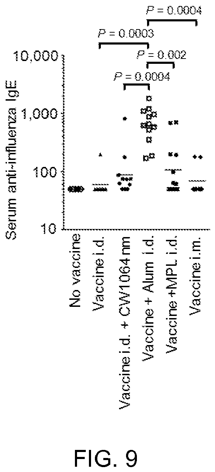

FIG. 9 is a graph showing the serum influenza-specific IgE titers in post-challenge status. Experimental and control groups: non-vaccination (n = 8), non-adjuvant (i.d. injection of influenza vaccine, n = 8), 1-min CW 1064 nm laser-treated (i.d. injection of influenza vaccine with the laser treatment, n = 11), i.d.-injected alum (n = 12), i.d.-injected MPL (n = 12), i.m.-injected groups (i.m. injection of influenza vaccine, n = 8), respectively.

### DETAILED DESCRIPTION

The ability of non-damaging visible light laser treatment of the skin to enhance immune responses to intradermal vaccination has been recently described in the literature (Onikienko SB, Zemlyanoy AB, Margulis BA, Guzhova IV, Varlashova MB, Gornostaev VS, Tikhonova NV, Baranov GA, Lesnichiy VV. (2007) Donosologiya (St. Petersburg) 2007; 1:32-54; Chen X, Kim P, Farinelli B, Doukas A, Yun SH, Gelfand JA, Anderson RR, Wu MX. (2010) PLoS One 5(10): e13776; WO 2009/044272). Laser illumination resulted in a significant enhancement of concomitant model vaccination in mice (Chen X, Kim P, Farinelli B, Doukas A, Yun SH, Gelfand JA, Anderson RR, Wu MX. (2010) PLoS One 5(10): e13776; WO 2009/044272). The same parameter of laser, when applied to the vaccination site, was also reported to have improved the response to influenza vaccination in patients who had failed to respond to a prophylactic influenza vaccine in the absence of laser usage (Onikienko et al. (2007)). Such laser treatments appear to alter the response of skin antigen-presenting cells to vaccine antigen, resulting in enhancement of serological response to vaccination in mice as well as the reported improvement in immune responses to influenza vaccination of non-responding patients (Onikienko et al. (2007); Chen et al. (2010)).

The present inventors have further developed this approach and have shown that continuous illumination of a skin vaccination site with a relatively low power (e.g., less than about 1 Watt) near-infrared (e.g., about 1064 nm) continuous wave (CW) laser enhanced antibody production to vaccination to a far greater extent than other laser approaches; illumination with a 1064nm CW laser lead to a 27-fold (27.2 ± 11.4) increase in antibody production to a laboratory vaccine (OVA at 6 weeks) (this can be compared to an 8-fold (7.9 ± 2.7) increase induced by a visible 532 nm laser).

As demonstrated herein, an influenza challenge study (experimental and control group size = 5-10) showed that at one minute infrared laser treatment before intradermal influenza vaccination reduced the vaccine dose needed to provide protection from lethal influenza challenge (protection was only observed in the 1 µg intradermal vaccine + laser group and 10 µg intramuscular vaccine only group compared to no protection in the 1 µg intradermal vaccine only and no vaccine groups).

The data presented herein further indicates that illumination at these doses does not damage tissue. This is in contrast to previous methods that (in some cases) purposefully induced tissue damage in an attempt to increase uptake (e.g., as described in US2003/0078499, US2001/0050083, and US 2004/0236268).

### Light Wavelength-Dependent Energy Absorption in the Skin Tissue

As described herein, a near-infrared 1064 nm laser can augment a laboratory vaccine in mice much more efficiently compared to 532 nm laser light. The precise mechanisms and responsible molecules for the adjuvant effect and signaling pathway for immune stimulation still remain elusive. Photobiological processes including vision in animals and photosynthesis in plants mainly utilize the wavelengths ranging from ultraviolet (UV, 300 nm) to near infrared (NIR, 900 nm), and the molecules responsible for these photoreceptions have been well studied and characterized for decades (. Similarly, the photons of the laser must be absorbed by some molecules to take any biological effects.

A chromophore is a molecule which imparts some decided color to the compound of which it is an ingredient, and governs mechanisms of laser-tissue interactions (Huang et al. (2009)). There are numerous chromophores in the skin possibly related to the laser adjuvant. Namely, cofactors such as flavin, heme, and metal ions represent absorption typically in the blue wavelength region of the visible spectrum; for example, hemoglobin, cytochrome c oxidase, cytochromes, flavins, flavoproteins, and porphyrins contain these moieties. These chromophores in the skin usually occur as one of two forms: conjugated pi electron systems and metal complexes, resulting in absorption of a certain spectrum of light (typically around 450 nm) (Karu TI. (2008) Photochem Photobiol 84(5): 1091-9; Huang YY, Chen AC, Carroll JD, Hamblin MR. (2009) Dose Response 7(4): 358-83; Karu T. (1999) J Photochem Photobiol B 49(1): 1-17; Niemz MH. (2007) Laser-Tissue Interactions: Fundamentals and Applications (Biological and Medical Physics, Biomedical Engineering) 308). Another class of dominant skin chromophores is melanin, which shows broad light absorption ranging from 400 to 700 nm. It absorbs ultraviolet radiation efficiently and transforms the energy into heat to protect DNA in skin cells (Meredith P, Riesz J. (2004) Photochem Photobiol 79(2): 211-6). Finally, a ubiquitous component of the biological system, water, is also a chromophore. The absorption coefficient, a measure showing the fraction of energy absorbed from a light beam, of pure water ranges from 0.03 to 0.06 cm⁻¹ between 760 - 1000 nm, and below 0.01 cm⁻¹ at < 700 nm of light beam, indicating that near-infrared and infrared content is strongly absorbed by water (Boulnois J. (1986) Lasers Med Sci 1(1): 47-66; Niemz MH. (2007) Laser-Tissue Interactions: Fundamentals and Applications (Biological and Medical Physics, Biomedical Engineering) 308). In short, light absorption in the skin tissue is determined by the chromophores and is therefore dependent on incoming light wavelength.

### Dendritic Cells in the Skin are a Target of Laser Light

Dendritic cells (DCs) are the most potent and versatile antigen-presenting cells (APCs), and play a pivotal role in adaptive immunity (Lanzavecchia A, Sallusto F. (2001) Curr Opin Immunol 13(3): 291-8; Trombetta ES, Mellman I. (2005) Annu Rev Immunol 23: 975-1028; Mellman I, Steinman RM. (2001) Cell 106(3): 255-8). DCs exhibit considerable heterogeneity in their anatomical location, surface phenotype, and functional properties, but skin-resident dendritic cell populations, namely Langerhans cells and dermal dendritic cells, position themselves in the skin and function as immune sentinels. DCs reside in the skin, and include Langerhans cells (LCs) and dermal DCs, which are the most potent APCs in the immune system (Nestle et al. (2009); Lanzavecchia A, Sallusto F. (2001) Curr Opin Immunol 13(3): 291-8;Trombetta ES, Mellman I. (2005) Annu Rev Immunol 23: 975-1028; Ruedl C, Koebel P, Bachmann M, Hess M, Karjalainen K. (2000) J Immunol 165(9): 4910-6). Upon encountering foreign antigen, they readily migrate to secondary lymphoid organs, particularly skin-draining lymph nodes (skin-DLNs), to stimulate naive or central memory T cells, and elicit potent and long-lasting immune responses (Randolph GJ, Inaba K, Robbiani DF, Steinman RM, Muller WA. (1999) Immunity 11(6): 753-61). Importantly, most of the current vaccine adjuvants and potential candidates have been considered to interact with DCs at the site of vaccine injection, and then help antigen uptake and migration of DLNs. This results in an increase in antigen presentation to T cells and a long-lasting immune response (Leroux-Roels G. (2010); Reed et al. (2009); Palucka K, Banchereau J, Mellman I. (2010) Immunity 33(4): 464-78). Interestingly, it has been demonstrated that low-power laser light induced cultured DC maturation in vitro (Chen AC-H, Huang YY, Sharma SK, Hamblin MR. (2010) SPIE: 756504-7), and that a moderately powered visible laser increased DC migration in the skin, resulting in an increase in antibody production in mice (Chen X, Kim P, Farinelli B, Doukas A, Yun SH, Gelfand JA, Anderson RR, Wu MX. (2010) A novel laser vaccine adjuvant increases the motility of antigen presenting cells. PLoS ONE 5(10): e13776). Thus, without wishing to be bound by theory, skin DCs are postulated to be important targets for the laser adjuvant.

### Vaccines and Vaccine Administration

The methods described herein can be used with any type of vaccine against a disease-causing pathogen, including, without limitation, bacterial, viral, and parasitic antigens. Exemplary vaccines include killed pathogen vaccines against influenza, cholera, bubonic plague, polio, hepatitis A, and rabies; attenuated vaccines, e.g., against yellow fever, measles, rubella, mumps, smallpox, typhoid, and Mycobacterium tuberculosis; Toxoid vaccines, e.g., against tetanus, diphtheria (including Crotalus atrox toxoid for rattlesnake bites); subunit vaccines, e.g., against Hepatitis B virus, virus-like particle (VLP) vaccine against human papillomavirus (HPV), and plague; and conjugate vaccines, e.g., the Haemophilus influenzae type B vaccine. In some embodiments, the vaccines may include any antigen, e.g., nucleic acid (e.g., RNA or DNA) or protein (e.g., polypeptide or peptide) antigens, and can be intact or denatured.

In the methods described herein, the vaccine is administered after the laser treatment, e.g., within 1, 2, 3, 4, 5, 6, 7, 8, 9, 10, 15, 30, 60, or 90 minutes after the laser treatment. Preferably, the vaccine is administered substantially immediately after the laser treatment, e.g., within 5 minutes.

The vaccine is administered intradermally, into or immediately adjacent to the skin that has been exposed to the laser treatment. The upper skin layers contain significant amounts of dendritic cells, thus making these tissues preferable targets of laser enhancement for vaccination. Intradermal vaccination has been used successfully in national vaccination campaigns including in Denmark and has been shown to produce equivalent seroconversion in two clinical trials (Nirmal S, Cherian T, Samuel BU, Rajasingh J, Raghupathy P, John TJ. Vaccine 1998;16(9):928-31; Mohammed AJ, AlAwaidy S, Bawikar S, Kurup PJ, Elamir E, Shaban MM, Sharif SM, van der Avoort HG, Pallansch MA, Malankar P, Burton A, Sreevatsava M, Sutter RW. N Engl J Med 2010;362(25):2351-9).

### Laser Adjuvants

In some embodiments, the methods described herein include the use of substantially non-damaging irradiation without pain or visible evidence of injury or inflammation (e.g., less than about 1 W) of a small (e.g., less than 0.5 cm²) area of the skin (e.g., with laser light), generally applied immediately prior to vaccination in the same area of the skin. One of skill in the art will appreciate that the parameters of the applied radiation can be varied while still ensuring that the irradiation (1) does not substantially damage the skin and (2) effectively enhances the immune response of a patient. Preferably the delivered dose is not painful.

In the present methods, the applied radiation is continuous-wave (CW) or pulsed corresponding to substantially a single optical wavelength. The central wavelength of the applied radiation (e.g., the wavelength at which the applied radiation is most intense) is generally selected to produce a desired variation in the immune response of a patient. In some embodiments, the central wavelength is between about 1000-1200 nm, e.g., between about 1040-1090, or between 1050-1080 nm, or between about 1060-1070 nm, e.g., about 1064 (e.g., plus or minus about 5, 10, 15, 20, or 25 nm, e.g., 1064 +/- 15 nm). In some embodiments, the lower limit of the possible range of central wavelength of the applied radiation is 1064 nm or less (e.g., 1050 nm or less, 1000 nm or less, 950 nm or less, 900 nm or less, but at least 850 nm). In certain embodiments, the upper limit of possible range of the central wavelength of the applied radiation is more than 1064 nm (e.g., 1100 nm or more, 1150 nm or more, but no more than 1200 nm).

In the methods described herein, the total dose of irradiation delivered to the patient is 25 J/cm² or more (e.g., 50 J/cm² or more, 75 J/cm² or more, 100 J/cm² or more, 200 J/cm² or more, 300 J/cm² or more, 400 J/cm² or more, 500 J/cm² or more , 600 J/cm² or more), up to about 1400 J/cm² or less (e.g., 1200 J/cm² or less, 1000 J/cm², 800 J/cm² or less).

In some embodiments, the average power, the beam size, and the exposure time can be selected to deliver a particular total dose of radiation to a patient to mediate the patient's immune response to one or more antigens while sparing the skin from substantial damage. Multiple combinations of average power, beam size, and exposure time can be used to deliver the same total dose of radiation, and these parameters can be varied to achieve other goals such as desired treatment times, reductions in physiological side-effects of the applied radiation (e.g., apoptosis), and degree of control over the boundary between exposed and non-exposed tissue.

In some embodiments, the laser energy may be delivered in a series of pulses where the pulse frequency optimizes heating of the skin. As the thermal relaxation time of the skin is 700 µsec to 1 msec and the effective relaxation time is about 40 times greater than the single-pulse relaxation time (Choi B, Welch AJ. Analysis of thermal relaxation during laser irradiation of tissue. Lasers Surg Med 2001;29(4):351-9), the thermal profile in the skin can be optimized when pulses are delivered a maximum of every 30-40 msec and the duration of individual pulses is equal to or less than this. In one aspect, the laser may operate in at pulse durations of 30-40 msec in a 50% duty cycle.

In another embodiment, individual pulse durations can be significantly shorter than 30-40 msec in order to control heat diffusion and optimize dose delivery, i.e., equal to or below the thermal relaxation time of 700-1,000 µsec. At the same time, such pulses will be of significantly greater duration than those that can cause intense photothermal or photoacoustic effects, i.e. above 1 µsec. In the methods described herein, the pulse durations may be 1-1,000 µsec with at least 30-40 msec between pulses.

Practioners of the art are familiar with strategies to combine pulse durations and pulse repetition in ways that optimize the delivery of laser energy to the skin while minimizing the possibility of damage or discomfort. Such a combination of pulse durations and pulse frequencies will be used in a way that shortens the time required to deliver an effective dose of light (i.e., 25-1400 J/cm2) in the shortest period of time while maintaining the exposed skin at temperature below the typical pain threshold (i.e about 42-43°C).

As one of skill in the art will appreciate, the total exposure time of the skin to the applied radiation can be selected to ensure that a clinically effective dose of radiation is delivered, while at the same time ensuring that no significant damage to the skin occurs. For example, the total exposure time can be 10 seconds or more (e.g., 30 seconds or more, 60 seconds or more, 120 seconds or more, 180 seconds or more, 240 seconds or more), with an upper limit of about 1 minute, 2 minutes, 3 minutes, or 4 minutes. Shorter exposures are preferred, e.g., 10-60 seconds.

The average power of the applied radiation can also be selected to ensure that a clinically effective dose of radiation is delivered without significant apoptosis. In some embodiments, the average power of the applied radiation is 0.5 W or more (e.g., 0.8 W or more, 1.0 W or more) and/or 5.0 W or less (e.g., 4.0 W or less, 2.0 W or less).

The applied radiation is typically delivered in a beam with a cross-sectional area of 0.5 cm² or less (e.g., 0.3 cm² or less, e.g., about 0.2 cm², or about 0.1 cm²) such that the power density of the applied radiation (irradiance) is 1.0 W/cm² or more (e.g., 0.1, 0.5, 1, 2, 3, 4, 5, 6, or 7 W/cm² or more), with an upper limit of 25 W/cm² or less (e.g., 20 W/cm² or less, 10 W/cm² or less). In some embodiments, the average irradiance is about 0.1-7 W/cm², e.g., about 1-5 W/cm², e.g., about 2-6 W/cm², e.g., about 5 W/cm².

In some embodiments, the cross-sectional intensity profile of the applied beam of radiation can be adjusted, for example, to ensure that exposed cells receive similar doses of radiation. In certain embodiments, for example, the cross-sectional intensity profile of applied beam can be substantially parabolic in shape. Using a parabolic beam of radiation can reduce simultaneous over-exposure of certain cells (e.g., near the center of the beam) and under-exposure of other cells (e.g., near the edges of the beam) that can occur with other beam shapes (e.g., certain Gaussian beams). To ensure that exposed cells receive similar doses of applied radiation, the cross-sectional intensity profile of the applied beam can be further adjusted such that the variations in intensity across the beam diameter are reduced still further. For example, the cross-sectional intensity profile of the beam can be adjusted (e.g., using spatial filtering optical elements) to match, as closely as possible, a square or "flat top" profile.

A variety of different devices can be used to deliver applied radiation to a patient. This disclosure, for purposes of clarity and brevity, describes the use of different laser sources for delivery of radiation. For example, there are existing low cost handheld laser systems that deliver the appropriate energies to enhance vaccine responses. Laser-based exposure can also be combined with microneedle-based or jet injector-based intradermal vaccine technologies to increase consistency of intradermal vaccination and identify the impact of laser treatment on reduction of antigen dose in these systems.

A variety of different laser sources can be used to produce radiation for purposes of this disclosure. In some embodiments, for example, Nd-based lasers (e.g., Nd:YAG and/or Nd:YVO₄ lasers) can be used to produce radiation. In certain embodiments, semiconductor lasers (e.g., lasers based on layered semiconductor materials including multiple quantum well lasers) can be used to produce radiation. Semiconductor lasers can, through judicious choice of materials, be selected to yield laser radiation in one or more desired wavelength bands.

Although these clinically safe and well-established laser technologies have been employed for various medical applications such as laser in situ keratomileusis (LASIK), lipolysis, hair removal, and photodynamic therapy (PDT) for decades (see, e.g., Aronoff BL. (1997) J Surg Oncol 64(1): 84-92; Krauss JM, Puliafito CA. (1995) Lasers Surg Med 17(2): 102-59; Houk LD, Humphreys T. (2007) Clin Dermatol 25(5): 434-42.; Niemz MH. (2007) Laser-Tissue Interactions: Fundamentals and Applications (Biological and Medical Physics, Biomedical Engineering) p.308), their use as an immunological adjuvant has heretofore not been explored. Infrared lasers can typically be easily housed in a device suitable for commercial development in a fairly straightforward way.

Other types of sources can also be used to produce suitable radiation. In particular, the radiation that is delivered to a patient need not be coherent radiation. Thus, a variety of other sources, including LED-based sources, laser diode-based sources, fluorescent sources, incandescent sources, and discharge-based sources, can yield radiation suitable for mediating immune. In some embodiments, the central wavelength of the radiation produced by these sources can be adjusted as desired. For example, the emission wavelengths of LED-based sources and laser diode-based sources can be controlled by the choices of materials used in fabricating the sources. In addition, multiple different source elements (e.g., multiple LEDs, multiple laser diodes) can be combined to yield broadband sources that can be selectively filtered as desired to yield radiation at a particular central wavelength for delivery to a patient. Similarly, broadband incandescent, fluorescent, and discharge-based sources can be filtered to yield radiation at a particular central wavelength using a variety of well-known optical filtering elements.

### EXAMPLES

The invention is further described in the following examples, which do not limit the scope of the invention described in the claims.

Unless otherwise specified below, the following methods were used in the following examples.

**Animals.** Seven-week-old female C57BL/6J mice were purchased from Jackson Laboratory. All mice were maintained in specific pathogen-free barrier facilities. All measurements were performed in a blinded manner, (to control or experimental groups), and all procedures were carried out following the Public Health Service Policy on Humane Care of Laboratory Animals and approved by the Institutional Animal Care and Use Committee of Massachusetts General Hospital.

**Lasers.** The lasers used in these experiments were Q-switched, diode-pumped solid state neodymium doped yttrium orthvanadate (Nd:YVO4) laser emitting light at 532 nm (UP6G, RMI Laser LLC, Lafayette, CO); an Nd:YVO4 laser emitting light at 1064 nm (UP10G, RMI Laser LLC, Lafayette, CO); or an Indium phosphide laser emitting light at 1470 nm (SemiNex Laser Co, Peabody, MA). The laser parameters are shown in Table 1.

**Table 1. Laser Parameters**

| Laser Parameters | Green Pulse | NIR* Pulse | NIR* CW | MIR** Pulse |
|---|---|---|---|---|
| Laser type | Nd:YVO₄ laser (UP6G, RMI Laser) | Nd:YVO₄ laser (UP10G, RMI Laser) | Nd:YVO₄ laser (UP10G, RMI Laser) | Indium phosphide (SemiNex Laser) |
| Wavelength | 532 nm | 1064 nm | 1064 nm | 1470 nm |
| Average power | 0.2W | 1.0W | 1.0W | 1.0W |
| Beam size | 5 mm (circle) | 5 mm (circle) | 5 mm (circle) | 5 mm (square) |
| Beam area | 0.2 cm² | 0.2 cm² | 0.2 cm² | 0.25 cm² |
| Beam profile | Parabolic+ | Parabolic+ | Parabolic+ | Flat top |
| Power density | 1.0 W/cm² | 5.0 W/cm² | 5.0 W/cm² | 4.0 W/cm² |
| Pulse duration | 10 nsec | 10 nsec | N.A. | 5 or 15 msec |
| Frequency | 10 kHz | 10 kHz | CW | 6 r 20 Hz |
| Exposure times (sec) | 60, 120, 240 | 60, 120, 240 | 60, 120, 240 | 300 |
| Doses (J/cm²) | 60, 120, 240 | 300, 600, 1200 | 300, 600, 1200 | 120 |

| | | | | |
|---|---|---|---|---|
| *NIR: near-infrared **MIR: mid-infrared +Parabolic beam is 50-100% more intense at center than at edge | | | | |

As shown in Table 1, these lasers produce 10 nanosecond (ns) - 15 millisecond (ms) duration laser pulses with a periodicity of 1-10 kHz. The average power is 200 - 1000 mW. The exposure area on the skin is approximately 0.2 cm². Exposure times for the mice were about 1-4 minutes. During laser exposure, the surface skin temperature was monitored with an infrared thermometer or a thermal imager to monitor heat-related skin damage.

**Laser illumination.** Experiments were performed using a neodymium-doped yttrium orthovanadate (Nd:YVO₄) laser emitting light at 1064 or 532 or nm. At 1064 nm, the laser produces either continuous wave (CW) or short, nanosecond duration laser pulses (PW) with a periodicity of 10 kHz, while at 532 nm it produces only PW. Average output powers were determined by a power meter for each illumination (S302C, Thorlabs). The beam profile was shaped to be relatively flat, with less than a 50% variation in beam intensity from center to edge. The laser spot size on the skin was approximately 5 mm, resulting in an exposure area of approximately 0.2 cm². One week after importation, mice hair was removed for illumination/vaccination. Mice were anesthetized with intramuscular injection of a cocktail of 90 mg Ketamine (Parke-Davis) and 9 mg Xylazine (Fermenta) per kg body weight. The backs of mice were then shaved and remaining hair was removed using a commercial hair remover (Nair, Church & Dwight Co.). The following day, mice were anesthetized and placed on a stage, and the shaved skin was illuminated with the laser. The skin on the back was divided into four areas on each mouse. One injection site was used for influenza vaccination. The skin temperature was monitored and measured during the procedure using an infrared thermal imager (FLIR Systems). The illuminated field on the skin was demarcated with a permanent marker immediately after each laser illumination to ensure a reproducible injection of vaccine solution in each illuminated spot.

**Skin damage study by visual inspection and histology.** To determine a non-tissue damaging dose of each laser parameter, the laser-treated skin was evaluated in a time-dependent manner by visual inspection and histology. Mice received CW or PW 1064 nm or PW 532nm laser illumination on the back skin for up to three min. Surface skin temperatures were monitored with an infrared thermal imager (FLIR Systems). Skin damage was evaluated at 0, 1, 2, and 4 days after illumination by visual inspection and histology. Maximum safe irradiances were considered to be those at which skin temperatures did not exceed 42° C for the duration of the exposure and for which no visible or microscopic skin damage was apparent at any of the post-exposure evaluation times. For skin histology, mice were perfusion fixed with 4% (w/v) paraformaldehyde at 0 (before the laser illumination), 3, 6, 24, 48, and 96 h after the laser illumination. The illuminated skin was excised and embedded in paraffin. Sections (5 µm thick) were H & E stained and examined by Dr. Roderick Bronson at Harvard Medical School for microscopic tissue damage and inflammatory responses.

**TUNEL staining.** TUNEL staining was performed on 5 µm-thick skin sections prepared as described in the previous section using the In situ Cell Death Detection Kit TMR Red (Roche Applied Science). Slides were imaged using confocal microscopy (Carl Zeiss LSM5 Pascal, Carl Zeiss North America).

**Ovalbumin administration.** Mice were injected intradermally with chromatographically purified ovalbumin (OVA, 10 µg in 10 µl saline, 4 spots, 40 µg in total, Worthington Biochemicals), which had been determined to contain less than 1.75 EUmg⁻¹ of endotoxin using the limulus amoeboid lysate assay (Cambrex Bio Science). Control mice received an intradermal injection of OVA alone without preceding laser illumination. Non-vaccinated control mice were sham-treated but did not receive OVA injection or laser illumination. The standard adjuvant, alum (Imject^{®} Alum, Thermo-Fisher) was used as a positive control. Blood and serum samples were drawn from the mice at 3, 6 and 12 weeks post-vaccination for the assessment of humoral immunity against OVA. The samples were collected via retro-orbital bleed under anesthesia with Ketamine and Xylazine. Control animals had a sham procedure in which they were anesthetized and shaved as above, but no laser was applied.

**Influenza administration.** Mice received a laser dose at a single vaccination site and mice were subsequently injected intradermally with 1 µg of whole inactivated influenza virus A/PR/8/34 (H1N1) (1 µg in 10 µl saline, Charles River Avian Vaccine Services) with or without 10 µg of monophosphoryl lipid A (VacciGrade™, a preclinical grade preparation of MPL, InvivoGen) or alum (Imject^{®} Alum, prepared so the final volume ratio of Imject Alum to immunogen is 1:1, Thermo-Fisher). Negative and positive controls included vaccine vehicle injection and intradermal or intramuscular vaccination with a 1 µg vaccine dose, respectively. Blood samples were drawn at 4 weeks post-vaccination or 4 days post-challenge with an intranasal application of live influenza viruses 4 weeks after vaccination to determine influenza-specific antibody production.

**ELISAs for quantitating anti-OVA and anti-influenza antibodies.** Immulon™ 2 HB Flat Bottom Plates (Thermo-Fisher) were coated overnight with 1 µg of the OVA at a concentration of 5 µgml⁻¹ or 0.2 µg of the inactivated influenza virus at 1 µgml⁻¹. Mouse serum samples diluted serially were added to the wells and incubated for 1 h on the coated and blocked plates. Bound immunoglobulins were detected with the appropriate horseradish peroxidase-conjugated secondary antibody (goat antibody to mouse IgG [1:10,000, Sigma-Aldrich]; rat antibody to mouse IgG1 [1:4,000, SouthernBiotech]; rat antibody to mouse IgG2b [1:500, SouthernBiotech]; goat antibody to mouse IgG2c [1:4,000, SouthernBiotech]; goat antibody to mouse IgA [1:1,000, Sigma-Aldrich]). At the end of the 1-h incubation, TMB substrate (Thermo Scientific Pierce 1-Step Ultra TMB, Thermo-Fisher) was added and the reaction was stopped with 2 N sulfuric acid. The reproducibility of the assay was ascertained by applying mouse anti-ovalbumin IgG (Sigma-Aldrich) or a hyperimmune mouse serum to influenza on each plate, we measured the absorption at 450 nm using an ELISA reader (TECAN Sunrise™ plate reader, TECAN). For titers of antibody to OVA, a statistically defined endpoint antibody titer was determined as previously described². For titers of antibody to influenza, a titer was designated as a serum dilution corresponding to a half the maximal activity.

**ELISAs for quantitating anti-influenza IgE antibody.** Immulon™ 2 HB Flat Bottom Plates (Thermo-Fisher) were coated overnight with 2 µg of the inactivated influenza virus at 10 µgml⁻¹. Mouse serum samples diluted serially were added to the wells and incubated for 1 h on the coated and blocked plates. Bound immunoglobulins were detected with the appropriate horseradish peroxidase-conjugated secondary antibody (rat antibody to mouse IgE [1:1,000, SouthernBiotech] followed by using a ELAST ELISA Amplification System (Perkin Elmer). Then TMB substrate (Thermo Scientific Pierce 1-Step Ultra TMB, Thermo-Fisher) was added and the reaction was stopped with 2 N sulfuric acid. The reproducibility of the assay was ascertained by applying a serum from mice immunized with inactivated influenza with alum adjuvant on each plate, we measured the absorption at 450 nm using an ELISA reader (TECAN Sunrise™ plate reader, TECAN). For titers of antibody to influenza, a statistically defined endpoint antibody titer was determined as previously described (Frey, A., et. al., A statistically defined endpoint titer determination method for immunoassays. J Immunol Methods 221, 35-41 (1998).

**Hemagglutination inhibition (HAI) titering for quantitating anti-influenza neutralizing antibodies.** Mouse sera was analyzed for a HAI titer by Charles River Avian Vaccine Services as described previously (Matsuoka, Y., et al., Curr Protoc Microbiol Chapter 15, Unit 15G 13 (2009); Szretter, K.J., et al., Curr Protoc Microbiol Chapter 15, Unit 15G 11 (2006)). All assays were performed in a manner that was blinded to the operator.

**Influenza challenge study.** Influenza A A/PR/8/34 (H1N1) virus used in this study was purchased from Charles River Avian Vaccine Services. Mice were anesthetized with Ketamine/Xylazine, and challenged intranasally with A/PR/8/34 at a dose of 1.5 x 10⁶ 50% egg infectious doses (EID₅₀) which is equivalent to 3 x 10³ 50% mouse lethal dose (MLD₅₀) in 30 µl saline. Survival and body weight were monitored for 14 days post-challenge. Mice showing a hunched posture, ruffled fur, greater than 20% body weight loss, or not eating or drinking were considered to have reached the experimental end point and were euthanized. MLD₅₀ titers were determined by inoculating groups of 10 mice intranasally with serial 10-fold dilutions of virus using the Reed-Muench formula as previously described (Matsuoka, Y., et al., Curr Protoc Microbiol Chapter 15, Unit 15G 13 (2009); Szretter, K.J., et al., Curr Protoc Microbiol Chapter 15, Unit 15G 11 (2006)).

**Virus titration in lung homogenate.** Four weeks after the initial immunization, mice were challenged with A/PR/8/34 as described above. Four days after the challenge, the mice were euthanized with carbon dioxide, and the lung was isolated and weighed. After adding PBS for a 10% (w/v) suspension, the isolated lung was homogenized using a disposable pestle system (Fisher Scientific). The 50% egg infectious dose (EID₅₀) was determined by serial titration of lung homogenate in eggs by Charles River Avian Vaccine Services. Briefly, serial dilutions of the tissue homogenates were inoculated into eggs, and EID₅₀ml⁻¹ values were calculated according to the method of Reed and Muench. All assays were performed in a manner that was blinded to the operator.

**Splenocyte stimulation and intracellular cytokine staining for anti-OVA and anti-influenza responses.** Four days after challenge as described above (four weeks after the initial immunization), splenocytes were harvested from individual mice, and red blood cells were lysed by using ACK buffer. Leukocytes were re-suspended in RPMI 1640 medium and incubated for 5 h in the presence of an inhibitor of Golgi function (Golgi plug, BD Biosciences), and 1 µgml⁻¹ of OVA or influenza MHC class I (OVA [257 - 264] SIINFEKL (SEQ ID NO. 1) or influenza A NP [366 - 374] ASNENMETM (SEQ ID NO. 2)), and II peptide (OVA [323-339] ISQAVHAAHAEINEAGR (SEQ ID NO. 3) or influenza A NP [311 - 325] QVYSLIRPNENPAHK (SEQ ID NO. 4)) for T cell stimulation. Multi-parameter surface staining for CD3ε, CD4, and CD8α, (CD3ε: clone 145-2C11; CD4: clone RM4-5; CD8α: clone 53-6.7, BD Biosciences) were carried out, followed by fixation, permeabilization in BD Cytofix/Cytoperm™ solution (BD Biosciences) according to the manufacturer's instructions and intracellular staining for IFN-γ or IL-17A or IL-5 (IFN-γ: clone XMG1.2; IL-17A: TC11-18H10 or IL-5: TRFK5, BD Biosciences). The positive control was stimulated with PMA/Ionomycin. Flow cytometry was run on a BD 4 Laser LSR II flow cytometer (BD Biosciences). Cell subpopulations, including OVA- or influenza-specific IFN-γ or IL-17A or IL-5 producing CD3+CD4+ T-helper cells and CD3+CD8+ T-cytotoxic cells were quantified as percentage in total viable cells by flow cytometry. Analysis was completed using BD FACSDiva™ 6.0 software (BD Biosciences) and gating was completed using a 'fluorescence minus one' (FMO) approach.

**Statistical analysis.** Statistical tests were performed under supervision of a biostatistician, Dr. Musie Ghebremichael in the Ragon Institute at Massachusetts General Hospital. Appropriate sample sizes and statistical methods were determined. The Mann Whitney U-test for the comparison of numerical values between 2 groups. The Kruskal-Wallis test was used for comparisons of more than three groups. Comparison of survival was done using the Gehan-Breslow-Wilcoxon test. Data were pooled from at least two independent experiments unless mentioned otherwise.

### Example 1. Non-Tissue Damaging Laser Parameters

A maximum non-tissue damaging dosage for the near-infrared ("NIR") and previously used visible lasers was established. Throughout this study, a neodymium doped yttrium orthvanadate (Nd:YVO4) laser was used. At 1064 nm, the laser produces either continuous wave (CW) or nanosecond duration laser pulses (PW) with a periodicity of 10 kHz, while at 532 nm it produces only PW. The beam profile was shaped to be relatively flat, with less than a 50% variation in beam intensity from center to edge. The laser spot size on the skin was approximately 5 mm, resulting in an exposure area of approximately 0.2 cm2. Tissue damage was evaluated by visual inspection and histology after laser illumination. Mice received continuous wave (CW) or nanosecond-pulsed (PW) 1064 nm or PW 532nm laser illumination on four areas of the shaved and depilated back skin for up to three min. Surface skin temperature was monitored with an infrared thermometer. (FIGs. 1A-C, left columns) Dose-temperature interactions of the PW 532 nm laser, PW 1064 nm laser, and CW 1064 nm laser in the mouse skin. 200mW (1 W/cm-2) for the PW 532 nm lasers and 1000 mW (5 Wcm-2) for the PW and CW 1064 nm are the maximum doses which do not reach the threshold for burn damage (42°C). n = 1-4 for each group. 5.0 Wcm-2 was identified as the maximum safe irradiance for both the 1064 nm PW and CW laser, and 1.0 Wcm-2 for PW 532 nm laser (FIGs. 1a-c) Images (FIGs. 1A-C, right columns) of mice back for visual inspection at 0 and 24 hours after cutaneous laser illumination. There was no visible damage, as evidenced by erythema, tissue edema or bruising, detected if the power is below 200mW for PW 532 nm lasers and 1000 mW for PW and CW 1064 nm. Arrow heads demonstrate visible skin damage. n =1-4 for each group. Representative images of each group are presented.

With the 532 nm pulsed laser (10 ns pulse width, 10 kHz), no significant damage or inflammation, and no apoptosis (FIG. 2A), was seen up to 200 mW (1000 mW/cm2). With the 1064 nm continuous wave (CW) or pulsed laser (10 ns pulse width, 10 kHz), no damage, apoptosis (FIG. 2B), or inflammation was seen up to 1000 mW (5000 mW/cm2). FIGs. 2A and 2B are a set of photomicrographs showing microscopic damage by the non-tissue damaging parameter of the laser adjuvant. Skin damage was evaluated at 0, 6, 24 hours after the laser illumination by histology. Representative time-course images of mouse skin histology with 4-min PW 532 nm (200 mW) and 4-min CW 1064 nm (1000 mW) laser illumination are shown. Skin damage was evaluated at 0, 6, 24 hours after the laser illumination by histology. The left 3 columns show images of TUNEL stained skin tissue. Five micron-thick paraffin block sections of the skin were stained using the In situ Cell Death Detection Kit TMR Red (Roche). Apoptotic cells were identified in red, nuclei were identified by counter staining using To-Pro-3 dye (blue). No damage was detected at any given time point. The bar indicates 50 µm. The right most columns of FIGs. 2A-B show images of hematoxylin-Eosin stained skin tissue. No damage or inflammatory response as evidenced by leukocyte and mononuclear cell-infiltration into the skin at any given time point was detected using this methodology. The bar indicates 50 µm. (a-c) n = 2-4 for each group. Representative images of each group are presented.

### Example 2. Evaluating Laser Adjuvant Using a OVA Laboratory Vaccine Model

To determine whether laser treatment could enhance the immune responses in mice, mice first received 1- or 4-min exposures to CW or PW 1064 nm or PW 532 nm laser at the subsequent vaccination site. After laser exposure, the mice received an intradermal (i.d.) injection of 40 µg of ovalbumin (OVA). (OVA, Worthington Biochemicals, 10 µg in 10 µl saline in 4 spots, 40 µg in total). Alum (Imject, Fisher-Thermo) was used as a positive control adjuvant.

The Experimental groups were as follows:
4 min laser + OVA (40 ug) i.d.
1 min laser + OVA (40 ug) i.d.
OVA (40 ug) + Alum i.d.
OVA (40 ug) + Alum i.m.
OVA (40 ug) i.d. only
Control (without vaccination)

Antibody titers to OVA were measured by ELISA for OVA-specific IgG at 3, 6, and 12 weeks after vaccination. Co-injection of alum with OVA served as positive controls. The antibody titer of the 4-min PW 532 nm laser group was significantly higher than that of non-adjuvant controls at 6 weeks (Fig. 3). However, its adjuvant effect was significantly smaller compared to alum-adjuvant controls. Both doses of PW 1064 nm laser with comparable parameters to the PW 532 nm laser groups showed significantly higher antibody titers than non-adjuvant controls at 6 weeks. Notably, the 1-min CW 1064 nm laser treatment induced the highest antibody titer which was equivalent to that of alum-adjuvant controls, and significantly higher than both the non-adjuvant controls and PW 532 nm laser group at all time points.

### Example 3. Effect of Laser Adjuvant In Murine Influenza Vaccination Model

To determine whether an infrared laser can enhance an immune response to vaccines and can confer appropriate protection against pathogens, a mouse model of influenza infection was used to demonstrate the ability of an infrared laser adjuvant to provide protective immunity against a pathogen. Mice were prepared and exposed to laser as described above, using the Nd:YVO₄ lasers at 532 nm (pulsed) and 1064 nm (continuous wave), and 1460 nm (pulsed). C57BL/6J mice received a laser dose at a single vaccination site and were injected i.d. with 1 µg of whole inactivated influenza virus A/PR/8/34 (Charles River Avian Vaccine Services, 1 µg in 10 µl saline) with or without laser illumination, monophosphoryl lipid A (MPL) or alum. Intramuscular (i.m.) vaccination with a 1 µg vaccine dose served as a positive control. Influenza-specific antibody production was analyzed by measuring titers of total IgG and its subclasses by ELISA and the influenza hemagglutination inhibition (HAI) assay. Samples were taken on day 28 after immunization and 4 days post-challenge with an intranasal application of live influenza virus (1.5 x 106 50% egg infectious doses per mouse) 4 weeks after vaccination.

FIG. 5A-C demonstrate serum influenza-specific IgG isotype titers in post-challenge status (4 days after challenge). FIG. 4A-C IgG subclass titers in pre-challenge status (4 weeks after vaccination). (FIG. 4A and 5A) IgG titers, (FIG. 4B and 5B) IgG1 titers, (FIG. 4C and 5C) IgG2c titers. Experimental and control groups: non-vaccination in post-challenge (FIG. 5A-C: n = 25) and pre-challenge status (FIG. 4A-C: n = 38), non-adjuvant (i.d. injection of influenza vaccine, FIG. 5A-C: n = 31; FIG. 4A-C: n = 49), 4-min PW 532 nm laser-treated (i.d. injection of influenza vaccine with the laser treatment, FIG. 5A-C: n = 13; FIG. 4A-C: n = 26), 1-min CW 1064 nm laser-treated (FIG. 5A-C: n = 34; FIG. 4A-C: n = 52), i.d.-injected alum (FIG. 5A-C: n = 12; FIG. 4A-C: n = 12), i.d.-injected MPL (FIG. 5A-C: n = 12; FIG. 4A-C: n = 12), i.m.-injected groups (i.m. injection of influenza vaccine, FIG. 5A-C: n = 16; FIG. 4A-C: n = 24), respectively. All procedures were carried out following the Public Health Service Policy on Humane Care of Laboratory Animals and approved by the Institutional Animal Care and Use Committee of Massachusetts General Hospital.

Post-challenge, the CW 1064 nm laser significantly augmented IgG, IgG1 and IgG2c titers compared to the non-adjuvant group. These data support the view that the CW 1064 nm laser treatment augments a mix of Th1 and Th2 immune response. The PW 532 nm laser group had more pronounced elevations in IgG2c titers than non-adjuvant controls, suggesting this parameter of laser augments a Th1-biased immune response. As shown in literature, alum induced Th2-biased immune response with Th2 cell-dependent antibody subclass (IgG1). IgG and IgG2c titers induced by the laser adjuvant are superior to two of licensed adjuvant (alum and MPL). The efficacy of alum adjuvant is higher than the laser adjuvant in IgG1 titer, but Th2-biased immune response by this adjuvant is connected to IgE-mediated hypersensitivity (see FIG. 9). Pre-challenge, the CW 1064 nm laser group significantly augmented IgG, IgG1 and IgG2c titers than non-adjuvant controls, but all the other differentials we saw in the post-challenge status are unclear at this particular time point. Together, the laser adjuvant is able to induce a comparable immune response as the licensed adjuvants.

Serum HAI titers, which a standard measurement of neutralizing antibody to influenza vaccination, tend to be higher in CW 1064 nm and PW 532 nm laser groups than in non-adjuvant control and i.d.-injected MPL groups, and also comparable to those of i.d.-injected alum and i.m.-injected groups. The results demonstrated in FIG. 6 suggest that the laser adjuvant is able to induce a comparable antibody immune response to a licensed adjuvant and conventional route of vaccination of influenza vaccine.

### Example 4. Effect of Laser Adjuvant on Systemic Cellular Immune Responses

Cell mediated immunity is critical for protection from influenza. To determine whether the laser elicits systemic cellular immune responses splenocytes were isolated and re-stimulated from influenza-challenged mice *ex vivo* with influenza peptides, and then assessed expression of cytokines in T-cell subpopulations by flow cytometry. Systemic cellular immune responses were determined from 5 h-splenocyte culture after isolation from vaccinated and unvaccinated mice at 4 days after challenge and re-stimulation with a nucleoprotein (NP) MHC class I influenza-specific peptide (NP 366-374). Influenza-specific CD4+IFN-γ+ T-cell as well as CD4+IL-5+ subpopulations induced with an MHC class II peptide were significantly increased in 1064 nm laser-treated group (FIG. 8a, b). These data are consistent with the view that the CW 1064 nm laser treatment augments a mix of Th1 and Th2 immune response, revealed by IgG subclass analysis (FIGs. 4 and 5). As shown in literature, MPL induced Th1-biased immune response as the influenza-specific CD4+IFN-γ+, but not CD4+IL-5+, T-cell subpopulation were significantly increased. Neither influenza-specific CD4+IL-17+ or CD8+ IFN-γ+ T-cell responses were significantly increased in any given experimental groups (FIG. 8c, d). These data demonstrate that CW 1064 nm laser enhances Th1 and Th2, but not Th17 or cytotoxic T cell immune responses to influenza vaccination. These data demonstrate that the laser treatment induced a robust systemic cellular immune response to an inactivated influenza vaccine without using any additional adjuvant.

FIG. 9A demonstrates Kaplan-Meier survival plots of vaccinated mice upon the lethal challenge. Survival of the CW 1064 nm laser-treated group was significantly better than that of the PW 532 nm laser-treated, non-adjuvant, and non-vaccination groups. The CW 1064 nm laser treatment consistently conferred better protective immunity against viral challenge than did the PW 532 nm laser, i.d.-vaccine alone, and no vaccine, determined by measuring survival time after viral challenge. (FIG. 9A).

FIG. 9B demonstrates the effect of the laser adjuvant on viral clearance. The 50% egg infectious dose (EID₅₀) was determined by serial titration of lung homogenate in eggs at 4 days after challenge. The CW 1064 nm laser group showed a marked decrease in lung viral titers of a factor of 10^{2.4}, 10^{1.7} and 10^{2.0} compared to the non-vaccination control, non-adjuvant control, and PW 532 nm laser-treated groups, respectively, on day 4 after challenge (FIG. 9B).

### Example 5. Effect of Laser Adjuvant In Murine Influenza Vaccination Model (IgE)

Serum influenza-specific IgE titers in post-challenge status (4 days after challenge). Mice were vaccinated by i.d. or i.m. injection of inactivated influenza virus (A/PR/8/34) with or without laser illumination, monophosphoryl lipid A (MPL) or alum. Experimental and control groups: non-vaccination (n = 8), non-adjuvant (i.d. injection of influenza vaccine, n = 8), 1-min CW 1064 nm laser-treated (i.d. injection of influenza vaccine with the laser treatment, n = 11), i.d.-injected alum (n = 12), i.d.-injected MPL (n = 12), i.m.-injected groups (i.m. injection of influenza vaccine, n = 8), respectively. (FIG. 9) Alum adjuvant is connected to IgE production and possibly hypersensitivity, but not laser adjuvant.

### Example 6. Laser Adjuvant for Enhancing Immune Response to Polio Vaccination

Though significant progress has been made under the Global Polio Eradication initiative led by the World Health Organization, poliomyelitis is still endemic to South Asia and Nigeria and is a dreaded disease of children in these areas. The oral polio vaccine (OPV) and the inactivated polio vaccine (IPV) have dramatically reduced the incidence of poliomyelitis over the past 50 years, but both vaccines have clear limitations in completing the immunization task. IPV requires four vaccinations and induces only an antibody-mediated immune response against the virus and not a protective effect against viral infection through the mucosal surface. The four vaccine requirement makes completion of vaccination protocol more difficult and results in incomplete disease eradication in the community. OPV induces mucosal immunity and confers good protection. However, the possibility of viral persistence and reversion to virulent wild-type raises safety concerns over OPV.

A continuous wave Nd:YVO4 laser emitting light at 1064 nm is used. The laser illuminates a small area of the skin (about 0.2 cm²) and generates a maximum of 2 Watts. An inactivated polio vaccine is used in established TgPVR21 mouse model or in an ICR mouse model, the background strain for the transgenic TgPVR21 mice used in polio vaccine protection studies (Nagata N et al., Virology 2004;321(1):87-100).

The maximum laser power that, during a one-minute exposure to the shaved back skin of ICR mice, does not raise skin surface temperature above 43°C (the threshold temperature for pain sensation in humans) is established. As described above, this dose level is non-damaging to mouse skin based on exhaustive visible and histological inspection. Groups of mice are treated with a series of exposures of up to about one minute at gradually decreasing laser power (e.g., 1.0 Watt, 0.8 W, 0.6 W, 0.4 W) followed immediately by intradermal vaccination with the inactivated poliovirus (1 µg in 10 µl saline). Blood samples are obtained at 0, 3, 6, and 12 weeks post-vaccination and polio-specific antibody (IgG) is quantitated using ELISA. The dose-response curve is mapped and the optimal laser dose for IPV immunization of ICR mice is identified. Then the geometric mean titers are compared between a two-dose course of optimal laser + intradermal IPV (0 and 3 weeks) and a three-dose course of intramuscular IPV alone (0, 3, and 6 weeks) where antigen dose of each vaccine is the same (1 µg). Blood is sampled at 0, 3, 6, and 12 weeks post-vaccination for quantitation of polio-specific antibody (IgG).

The established TgPVR21 mouse model is used to demonstrate the ability of the laser + vaccine regimen to provide protective immunity against poliovirus infection (Nagata N et al., Virology 2004;321(1):87-100). The ability of the laser to induce mucosal immune response to IPV is measured.

It is expected that the proper dose of infrared laser light, at power and dose levels that will be non-damaging and painless in humans, will similarly enhance humoral immune response to intradermal immunization with IPV to a degree that makes reduction in the number of booster vaccines practical, making it easier to achieve protective IPV vaccination.

### Example 7 Laser adjuvant and Dendritic Cell Recruitment, Saturation, and Migration

The adequate and timely trafficking of skin DCs to lymph nodes (LNs), where DCs interact with T and B cells and mediate antigen presentation through lymphatic vessels in the skin, is crucial for the execution of their functions (Randolph GJ, Angeli V, Swartz MA. (2005) Dendritic-cell trafficking to lymph nodes through lymphatic vessels. Nat Rev Immunol 5(8): 617-28). The directed DC migration is mediated by selective expression of chemokine receptors and by the generation of specific chemokines in immune organs (Trombetta ES, Mellman I. (2005) Annu Rev Immunol 23: 975-1028; Randolph GJ, Angeli V, Swartz MA. (2005) Nat Rev Immunol 5(8): 617-28;Sallusto F, Palermo B, Lenig D, Miettinen M, Matikainen S, Julkunen I, Forster R, Burgstahler R, Lipp M, Lanzavecchia A. (1999) Eur J Immunol 29(5): 1617-25; Vecchi A, Massimiliano L, Ramponi S, Luini W, Bernasconi S, Bonecchi R, Allavena P, Parmentier M, Mantovani A, Sozzani S. (1999) J Leukoc Biol 66(3): 489-94). In the non-stimulated skin, LCs and dermal DCs are motile to acquire and process foreign antigens. They express the chemokine receptors CCR1, CCR2, CCR5, and CCR6, as well as CXCR3 and CXCR4 (Trombetta ES, Mellman I. (2005) Annu Rev Immunol 23: 975-1028; Vecchi A, Massimiliano L, Ramponi S, Luini W, Bernasconi S, Bonecchi R, Allavena P, Parmentier M, Mantovani A, Sozzani S. (1999) J Leukoc Biol 66(3): 489-94). DCs down-regulate most chemokine receptors during their maturation process, with the exception of CXCR4 and CCR7, upon exposure to inflammatory signals such as chemokines or cytokines (e.g., IL-8 or TNF-alpha) (Luster AD, Weinshank RL, Feinman R, Ravetch JV. (1988) J Biol Chem 263(24): 12036-43; Proudfoot AE. (2002) Nat Rev Immunol 2(2): 106-15) and foreign antigens (e.g., LPS or TLR ligands) (De Smedt T, Pajak B, Muraille E, Lespagnard L, Heinen E, De Baetselier P, Urbain J, Leo O, Moser M. (1996) J Exp Med 184(4): 1413-24; Stockwin LH, McGonagle D, Martin IG, Blair GE. (2000) Immunol Cell Biol 78(2): 91-102). CXCR4 is highly expressed on migrated skin DCs in vivo (Kabashima K, Shiraishi N, Sugita K, Mori T, Onoue A, Kobayashi M, Sakabe J, Yoshiki R, Tamamura H, Fujii N, Inaba K, Tokura Y. (2007) Am J Pathol 171(4): 1249-57) and mediates directional migration (Kabashima K, Shiraishi N, Sugita K, Mori T, Onoue A, Kobayashi M, Sakabe J, Yoshiki R, Tamamura H, Fujii N, Inaba K, Tokura Y. (2007) Am J Pathol 171(4): 1249-57; Humrich JY, Humrich JH, Averbeck M, Thumann P, Termeer C, Kampgen E, Schuler G, Jenne L. (2006) Immunology 117(2): 238-47; Ouwehand K, Santegoets SJ, Bruynzeel DP, Scheper RJ, de Gruijl TD, Gibbs S. (2008) Eur J Immunol 38(11): 3050-9).

To identify cytokines induced by laser illumination, qPCR analysis was performed on laser illuminated skin tissue. Interleukin-6 (IL-6) and CCR7, which are critical for skin-resident dendritic cell maturation and migration, were highly upregulated 6 hours after the laser treatment among 160+ cytokines and their examined receptors (IL-6: 6.1 fold; CCR7: 4.4 fold compared to non-illuminated skin tissue, n = 3 for each gene). Surprisingly, laser illumination does not upregulate classical inflammatory cytokines such as TNFa, IL1b, or IFN-gamma. IL6 is expressed in the mid-epidermis layer (presumably keratinocytes), most substantially at 6 hours post-illumination.

Immunofluorescence analysis was performed to determine the spatial and temporal distribution of IL-6. The skin of the mouse back illuminated with the laser induced IL-6 expression in the mid-epidermal layer, adjacent to Langerhans cells (skin-resident dendritic cells). The expression level peaked at 6 hours after the laser application.

Since the magnitude of immune response depends on the amount of DCs migrating into secondary lymphoid organs (Leroux-Roels G. (2010)Reed et al. (2009); Palucka K, Banchereau J, Mellman I. (2010) Immunity 33(4): 464-78), cytokines induced by the laser illumination in the skin may play a critical role in the initiation of a skin immune response

Transcriptome analysis by real-time quantitative PCR on the laser-treated mouse skin demonstrated that laser illumination (both PW 532 and CW 1064nm) selectively upregulates a consistent set of genes which are known to associate with dendritic cell recruitment and activation including ccl2, ccl17, cc120, cxcl1, cxc113, ccr7, ptgs2 (COX-2), and IL6 (data not shown). The CW1064 nm laser illumination does not upregulate classical inflammatory cytokines such as TNF-alpha, IL1-beta, IFN-gamma, but PW 532 does. Additionally, histology revealed accumulation of CD11c+ dendritic cells, which are the most versatile antigen presenting cells in the skin, in the spot where CW 1064 nm laser was applied without inducing any tissue damage. Without wishing to be being bound by theory, it is presently believed that the expression of a cocktail of cytokines induced by the laser adjuvant may be associated to recruitment, migration and activation of dendritic cells and the adjuvant effect.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. An antigen for use in a method of enhancing a protective immune response to the antigen in a subject, the method comprising:
exposing the skin of the subject to radiation at a wavelength of between 1050 nm and 1080 nm, wherein the radiation is in a total dose of near-infrared radiation of between 25 J and 1200 J; and
administering to the subject a dose of the antigen, wherein the antigen is administered intradermally, into or immediately adjacent to, the skin exposed to the irradiation;
**characterised in that**:
the radiation is a continuous-wave radiation.

2. The antigen for the use of claim 1, wherein the continuous-wave radiation has a wavelength of 1064 nm

3. The antigen for the use of any one of the preceding claims, wherein the radiation comprises laser radiation; preferably the laser being a neodymium-doped yttrium aluminum garnet (Nd:YAG) laser.

4. The antigen for the use of any one of the preceding claims, wherein the antigen comprises nucleic acid or protein.

5. The antigen for the use of any one of the preceding claims, wherein the antigen is viral, bacterial, fungal, unicellular or multicellular parasite or a portion thereof.

6. The antigen for the use of claim 5, wherein the antigen is an influenza virus or a portion thereof.

7. The antigen for the use of claim 5, wherein the antigen is from polio, hepatitis A, hepatitis B, hepatitis C, influenza, tuberculosis (Bacillus Calmette-Guérin (BCG)), measles, tetanus toxoid, tick-bourne encepalisis, yellow fever, diphtheria-tetenus-pertussis, human papilloma virus, multivalent meningitis conjugate vaccine, pneumococcal conjugate vaccine or rabies pathogen. P

8. The antigen for the use of any one of the preceding claims, wherein a total exposure time of the subject is between 10 s and 300 s.

9. The antigen for the use of any one of the preceding claims, wherein a cross-sectional intensity profile of the radiation is substantially parabolic in shape.

10. The antigen for the use of claims 1-2 or 4-9, wherein the radiation comprises radiation produced by a non-laser source.

11. The antigen for the use of any one of the preceding claims, wherein the average irradiance is 1 W/cm2.

12. The antigen for the use of any one of the preceding claims, wherein the average irradiance is between 1 W/cm2 and 5 W/cm2.

## Patentansprüche

1. Antigen zur Verwendung in einem Verfahren zur Steigerung einer schützenden Immunantwort auf das Antigen in einem Subjekt, wobei das Verfahren Folgendes umfasst:
Exposition der Haut des Subjekts an Dauerstrichstrahlung mit einer Wellenlänge zwischen 1050 nm und 1080 nm, worin die Dauerstrichstrahlung in einer Gesamtdosis nahinfraroter Strahlung zwischen 25 J und 1200 J vorliegt; und
Verabreichung an das Subjekt einer Dosis des Antigens, worin das Antigen intradermal in die oder unmittelbar neben der Haut, die der Bestrahlung ausgesetzt ist, verabreicht wird.

2. Antigen zur Verwendung nach Anspruch 1, worin die Dauerstrichstrahlung eine Wellenlänge von 1064 nm aufweist.

3. Antigen zur Verwendung nach einem der vorhergehenden Ansprüche, worin die Strahlung Laserstrahlung umfasst; der Laser vorzugsweise ein Neodym-dotierter Yttrium-Aluminium-Granat(Nd:YAG)-Laser ist.

4. Antigen zur Verwendung nach einem der vorhergehenden Ansprüche, worin das Antigen Nukleinsäure oder Protein umfasst.

5. Antigen zur Verwendung nach einem der vorhergehenden Ansprüche, worin das Antigen ein viraler, bakterieller, fungaler, einzelliger oder mehrzelliger Parasit oder ein Teil davon ist.

6. Antigen zur Verwendung nach Anspruch 5, worin das Antigen ein Influenzavirus oder ein Teil davon ist.

7. Antigen zur Verwendung nach Anspruch 5, worin das Antigen aus Polio, Hepatitis A, Hepatitis B, Hepatitis C, Influenza, Tuberkulose (Bacillus Calmette-Guerin (BCG)), Masern, Tetanus-Toxoid, Zeckenencephalitis, Gelbfieber, Diphtherie-Tetanus-Pertussis, Humanpapillomvirus, polyvalentem Meningitis-Konjugat-Impfstoff, Pneumokokken-Konjugat-Impfstoff oder Tollwut-Pathogen stammt.

8. Antigen zur Verwendung nach einem der vorhergehenden Ansprüche, worin eine insgesamte Expositionszeit des Subjekts zwischen 10 s und 300 s beträgt.

9. Antigen zur Verwendung nach einem der vorhergehenden Ansprüche, worin ein Querschnitts-Intensitätsprofil der Strahlung im Wesentlichen parabolförmig ist.

10. Antigen zur Verwendung nach Anspruch 1-2 oder 4-9, worin die Strahlung durch eine Nichtlaserquelle erzeugte Strahlung umfasst.

11. Antigen zur Verwendung nach einem der vorhergehenden Ansprüche, worin die durchschnittliche Bestrahlungsstärke 1 W/cm² beträgt.

12. Antigen zur Verwendung nach einem der vorhergehenden Ansprüche, worin die durchschnittliche Bestrahlungsstärke zwischen 1 W/cm² und 5 W/cm² beträgt.

## Revendications

1. Antigène destiné à être utilisé dans un procédé de renforcement d'une réponse immunitaire de protection à l'antigène chez un sujet, le procédé consistant à:
exposer la peau du sujet à un rayonnement à onde continue à une longueur d'onde comprise entre 1050 nm et 1080 nm, le rayonnement à onde continue étant dans une dose totale de rayonnement proche de l'infrarouge comprise entre 25 J et 1200 J ; et
administrer au sujet une dose de l'antigène, l'antigène étant administré par voie intradermique, dans la peau ou à proximité immédiate de la peau exposée au rayonnement.

2. Antigène destiné à être utilisé selon la revendication 1, dans lequel le rayonnement à onde continue a une longueur d'onde de 1064 nm.

3. Antigène destiné à être utilisé selon l'une quelconque des revendications précédentes, dans lequel le rayonnement consiste en un rayonnement laser ; le laser étant de préférence un laser au grenat d'yttrium et d'aluminium dopé au néodyme (Nd:YAG).

4. Antigène destiné à être utilisé selon l'une quelconque des revendications précédentes, l'antigène comprenant un acide nucléique ou une protéine.

5. Antigène destiné à être utilisé selon l'une quelconque des revendications précédentes, dans lequel l'antigène est un parasite viral, bactérien, fongique, unicellulaire ou multicellulaire ou une partie correspondante.

6. Antigène destiné à être utilisé selon la revendication 5, l'antigène étant un virus grippal ou une partie correspondante.

7. Antigène destiné à être utilisé selon la revendication 5, l'antigène provenant d'une poliomyélite, d'une hépatite A, d'une hépatite B, d'une hépatite C, d'une grippe, d'une tuberculose (Bacille de Calmette et Guérin (BCG)), d'une rougeole, d'un toxoïde tétanique, d'une encéphalite à tiques, d'une fièvre jaune, d'une diphtérie-tétanos-poliomyélite, d'un papillomavirus humain, d'un vaccin conjugué multivalent contre la méningite, d'un vaccin conjugué contre le pneumocoque ou d'un pathogène de la rage.

8. Antigène destiné à être utilisé selon l'une quelconque des revendications précédentes, dans lequel une durée d'exposition totale du sujet est comprise entre 10 s et 300 s.

9. Antigène destiné à être utilisé selon l'une quelconque des revendications précédentes, dans lequel le profil d'intensité en coupe du rayonnement est pratiquement de forme parabolique.

10. Antigène destiné à être utilisé selon les revendications 1 et 2 ou 4 à 9, dans lequel le rayonnement consiste en un rayonnement produit par une source non laser.

11. Antigène destiné à être utilisé selon l'une quelconque des revendications précédentes, dans lequel l'irradiation moyenne est de 1 W/cm2.

12. Antigène destiné à être utilisé selon l'une quelconque des revendications précédentes, dans lequel l'irradiation moyenne est comprise entre 1 W/cm2 et 5 W/cm2.
